# EUROPÄISCHE PATENTSCHRIFT

(11) **EP 0 904 050 B1**
(45) Veröffentlichungstag und Bekanntmachung des Hinweises auf die Patenterteilung: **30.01.2002**
(21) Anmeldenummer: 97919348.9
(22) Anmeldetag: 12.04.1997
(51) Int. Cl.: A61K 7/48, A61K 47/14

(54) **VERWENDUNG VON MILCHSÄUREESTERN**
USE OF LACTIC ACID ESTERS
UTILISATION D'ESTERS D'ACIDE LACTIQUE

(30) Priorität: 20.04.1996 DE 19615777
(43) Veröffentlichungstag der Anmeldung: 31.03.1999
(73) Patentinhaber: Cognis Deutschland GmbH, 40589 Düsseldorf (DE)
(72) Erfinder: WADLE, Armin, D-40724 Hilden (DE); ANSMANN, Achim, D-40699 Erkrath (DE); VON KRIES, Rainer, D-89257 Illertissen (DE)
(86) Internationale Anmeldenummer: EP9701835
(87) Internationale Veröffentlichungsnummer: WO9739731

(56) Entgegenhaltungen:
- CH-A- 443 565
- DE-A- 4 412 081
- US-A- 5 221 286

## Beschreibung

### Gebiet der Erfindung

Die Erfindung betrifft die Verwendung von Milchsäureestern mit definiertem Gehalt an gebundener Milchsäure als O/W-Emulgatoren für die Herstellung von kosmetischen und pharmazeutischen Zubereitungen.

### Stand der Technik

Milchsäureester auf fettchemischer Basis, d.h. Umsetzungsprodukte von Milchsäure mit Fettsäuren, Fettalkoholen oder Glyceriden, stellen bekannte O/W-Emulgatoren des Stands der Technik dar, die wegen ihrer besonders vorteilhaften dermatologischen Verträglichkeit eine besondere Bedeutung bei der Formulierung von kosmetischen und pharmazeutischen Zubereitungen besitzen, bei denen besonders hohe Anforderungen an die hautkosmetische Verträglichkeit gestellt werden (siehe z.B. CH-A-443 565, DE-A-44 12 081 und US-A-5 221 286). Die Verträglichkeit der Milchsäureester, die häufig auch als Lactate bezeichnet werden, ist dabei so hoch, daß sie auch als Lebensmittelemulgatoren Verwendung finden.

Gleichwohl ist das Emulgiervermögen der Milchsäureester des Handels nicht befriedigend, da die auf dieser Basis hergestellten O/W-Emulsionen die Tendenz zeigen, im Verlauf der Lagerung einzudicken und sich zu entmischen. Der optische Eindruck derartiger Emulsionen ist zuweilen so unbefriedigend, daß der Einsatz von Milchsäureestern für das Herstellen spezieller, besonders eleganter Emulsionen trotz der hohen dermatologischen Vorteile bestenfalls eingeschränkt in Frage kommt.

Die Aufgabe der Erfindung hat demnach darin bestanden, diesem Mangel abzuhelfen und neue Milchsäureester zur Verfügung zu stellen, die die bekannte dermatologische Verträglichkeit mit einer erhöhten Emulgierleistung und einer verbesserten Emulsionsstabilität verbinden.

### Beschreibung der Erfindung

Gegenstand der Erfindung ist die Verwendung von Milchsäureestern der Formeln **(Ia), (Ib)** und/oder **(Ic),** 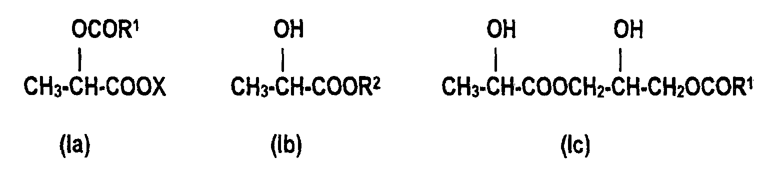 in denen R¹CO für einen linearen oder verzweigten, gesättigten und/oder ungesättigten Acylrest mit 6 bis 22 Kohlenstoffatomen, R² für einen Alkyl- und/oder Alkenylrest mit 6 bis 22 Kohlenstoffatomen und X für ein Alkali- und/oder Erdalkalimetall steht, als O/W-Emulgatoren für die Herstellung von kosmetischen und/oder pharmazeutischen Zubereitungen, wobei die Emulgatoren einen Gehalt an gebundener Milchsäure im Bereich von 10 bis 40 und vorzugsweise 20 bis 35 Gew.-% aufweisen.

Im Rahmen umfangreicher Untersuchungen hat die Anmelderin festgestellt, daß der Gehalt an gebundener Milchsäure in den Estern für die gewünschten anwendungstechnischen Eigenschaften kritisch ist. Überraschenderweise wurde gefunden, daß die für die Verwendung als Lebensmittelemulgatoren an sich bekannten Milchsäureester bei Einsatz in Kosmetik und Pharmazie ein Optimum hinsichtlich Emulgiervermögen und Stabilität aufweisen, wenn sie einen definierten Gehalt an gebundener Milchsäure im Bereich von 10 bis 40 Gew.-% - bezogen auf die Ester - enthalten. Insbesondere Ester mit einem höheren Milchsäuregehalt - wie dies bei Produkten des Handels der Fall ist - führen dazu, daß die auf dieser Basis hergestellten Emulsionen allmählich eindicken und sich dabei entmischen. Dieser unerwünschte Effekt wird bei erfindungsgemäßer Verwendung der verbesserten Milchsäureester zuverlässig vermieden.

### Milchsäureester

Milchsäureester stellen bekannte Stoffe des Stands der Technik dar, die in der Regel durch Umsetzung von Milchsäure mit Fettsäuren, Fettalkoholen oder Glycerin erhalten werden. In Abhängigkeit der Menge an Einsatzstoffen läßt sich ein definierter Gehalt an gebundener Milchsäure in den Veresterungsprodukten einstellen [vgl. A.M.Chaudhry et al. in **Pak.J.Sci.Res. 33, 344 (1990).]**

Im Sinne der Erfindung kommen als Milchsäureester, die der Formel (**la**) folgen, Veresterungsprodukte von Milchsäure mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen in Frage, die in Form ihrer Alkali - und/oder Erdalkalisalze vorliegen können. Bevorzugt ist der Einsatz von Milchsäureestern der Formel (**la**), bei denen sich der Acylrest R¹CO von Fettsäuren mit 16 bis 22 Kohlenstoffatomen ableitet, wie beispielsweise das Natriumsalz des Stearoyllactats (Sodium Stearoyl Lactate).

Neben Estern, die durch Acylierung der Hydroxylgruppe der Milchsäure erhalten werden, kommen auch Ester in Frage, die der Formel **(Ib)** folgen und Veresterungsprodukte der Milchsäure mit Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen darstellen. Bevorzugt ist der Einsatz von Milchsäureestern der Formel **(Ib),** bei denen sich der Alkylrest R² von Fettalkoholen mit 16 bis 22 Kohlenstoffatomen ableitet, wie beispielsweise Milchsäurestearylester.

Als dritte Gruppe von Milchsäureestern kommen Partialglyceride der Formel **(Ic)** in Frage, die beispielsweise durch Veresterung der Milchsäure mit technischen Partialglyceriden erhalten werden. Dabei entstehen in der Regel technische Gemische, in denen auch Estolide und Lactide - dies trifft auch für die Ester der Formeln **(la)** und **(Ib)** zu - enthalten sein können. Die Partialglyceride ihrerseits können Mono- und/oder Diester des Glycerins mit Fettsäuren mit 6 bis 22 und vorzugsweise 12 bis 18 Kohlenstoffatomen darstellen.

Allen diesen Estertypen ist gemeinsam, daß sie in einer besonders bevorzugten Ausführungsform der Erfindung einen Gehalt an gebundener Milchsäure im Bereich von 20 bis 35 Gew.-% aufweisen.

### Kosmetische und/oder pharmazeutische Zubereitungen

Die erfindungsgemäß einzusetzenden Milchsäureester stellen O/W-Emulgatoren dar und eignen sich dementsprechend zur Herstellung von kosmetischen und/oder pharmazeutischen Zubereitungen wie beispielsweise Haar- und Hautpflegemitteln, als da sind Haarshampoos, Haarlotionen, Duschbädern, Körperlotionen, Pflegecremes und Salben.

Die Mittel können als weitere Bestandteile Tenside, Ölkörper, Co-Emulgatoren, Überfettungsmittel, Konsistenzgeber, Verdickungsmittel, Kationpolymere, Siliconverbindungen, Fette, Wachse, Stabilisatoren, biogene Wirkstoffe, Filmbildner, Konservierungsmittel, Farb- und Duftstoffe enthalten.

Typische Beispiele für geeignete **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Ethercarbonsäuren, Alkyloligoglucoside, Fettsäureglucamide, Alkylamidobetaine und/oder vorzugsweise pflanzliche Proteinfettsäurekondensate.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe in Betracht.

Als **Co-Emulgatoren** können nichtionogene, ampholytische und/oder zwitterionische grenzflächenaktive Verbindungen verwendet werden, die sich durch eine lipophile, bevorzugt lineare Alkyl - oder Alkenylgruppe und mindestens eine hydrophile Gruppe auszeichnen. Diese hydrophile Gruppe kann sowohl eine ionogene als auch eine nichtionogene Gruppe sein. Nichtionogene Emulgatoren enthalten als hydrophile Gruppe z. B. eine Polyolgruppe, eine Polyalkylenglycolethergruppe oder eine Kombination aus Polyol- und Polyglycolethergruppe. Bevorzugt sind solche Mittel, die nichtionogene Tenside aus mindestens einer der folgenden Gruppen enthalten:
(a1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(a2) C_{12/18}-Fettsäuremono- und -diester von Anlagerungsprodukten von 1 bis 30 Mol Ethylenoxid an Glycerin;
(a3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(a4) Alkylmono- und -oligoglycoside mit 8 bis 22 Kohlenstoffatomen im Alkylrest und deren ethoxylierte Analoga;
(a5) Anlagerungsprodukte von 15 bis 60 Mol Ethylenoxid an Ricinusöl und/oder gehärtetes Ricinusöl;
(a6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl* Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampho-lytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkyl-gruppe. Besonders bevorzugte ampholytische Tenside sind das N-Kokosalkylaminopropionat, das Kokosacylaminoethylaminopropionat und das C_{12/18}-Acylsarcosin.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdikkungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationischen Cellulosederivate, kationischen Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Pflanzenextrakte und Vitaminkomplexe zu verstehen. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

### Beispiele

Zwei Emulsionen bestehend aus 3 Gew.-% Sodium Stearoyl Lactylate, 5 Gew.-% Glyceryl Stearate und 10 Gew.-% Decyl Oleate (Wasser ad 100 Gew.-%) wurden über 8 Wochen bei 20°C gelagert und dabei auf ihre Viskosität (Brookfield RVF-Viskosimeter, 20°C, Spindel 1, 20 Upm), Stabilität und Emulgierleistung untersucht. Dabei wurde im erfindungsgemäßen Beispiel ein Milchsäureester mit 25 Gew.-% gebundener Milchsäure, im Vergleichsbeispiel der gleiche Ester mit einem Gehalt von 42 Gew.-% gebundener Milchsäure (Handelsprodukt) eingesetzt. Die Ergebnisse sind in Tabelle 1 zusammengefaßt.

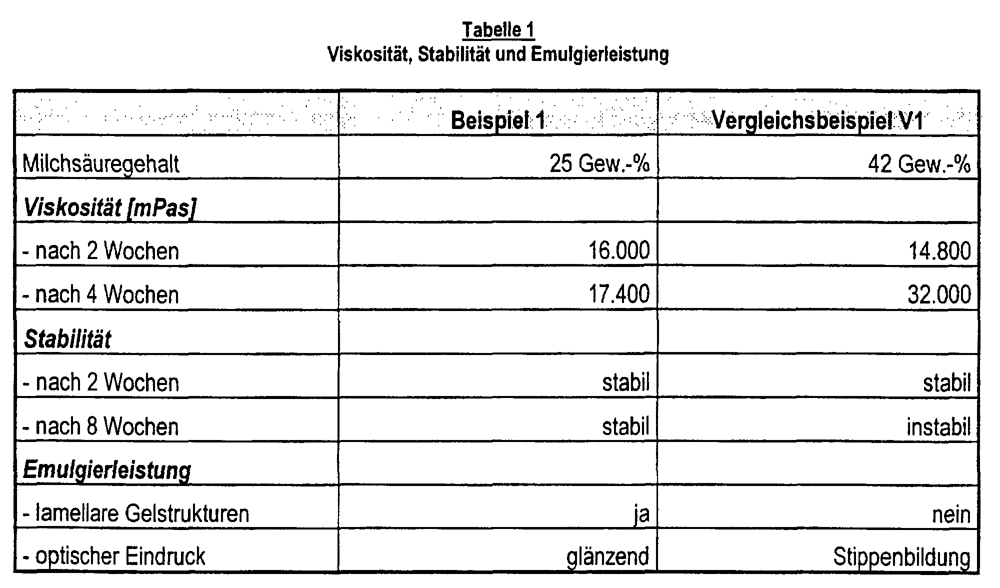

Man erkennt, daß stabile, glänzende, niedrigviskose Emulsionen nur dann erhalten werden, wenn man den Gehalt an gebundener Milchsäure im Sinne der Erfindung begrenzt.

## Patentansprüche

1. Verwendung von Milchsäureestern der Formeln **(la), (Ib)** und/oder **(Ic),** 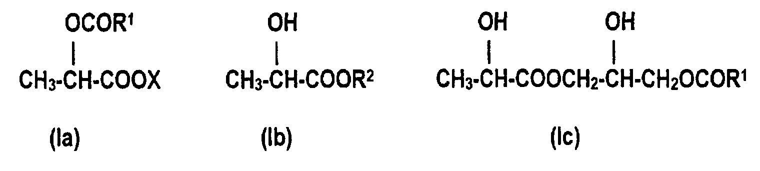 in denen R¹CO für einen linearen oder verzweigten, gesättigten und/oder ungesättigten Acylrest mit 6 bis 22 Kohlenstoffatomen, R² für einen Alkyl- und/oder Alkenylrest mit 6 bis 22 Kohlenstoffatomen und X für ein Alkali- und/oder Erdalkalimetall steht, als O/W-Emulgatoren für die Herstellung von kosmetischen und/oder pharmazeutischen Zubereitungen, **dadurch gekennzeichnet, daß** die Emulgatoren einen Gehalt an gebundener Milchsäure im Bereich von 10 bis 40 Gew.-% aufweisen.

2. Verwendung nach Anspruch 1, **dadurch gekennzeichnet, daß** man Milchsäureester der Formel **(Ia)** einsetzt, bei denen sich der Acylrest R¹CO von Fettsäuren mit 16 bis 22 Kohlenstoffatomen ableitet.

3. Verwendung nach den Ansprüchen 1 und 2, **dadurch gekennzeichnet, daß** man Milchsäureester der Formel **(Ib)** einsetzt, bei denen sich der Alkylrest R² von Fettalkoholen mit 16 bis 22 Kohlenstoffatomen ableitet.

4. Verwendung nach den Ansprüchen 1 bis 3, **dadurch gekennzeichnet, daß** man Milchsäureester einsetzt, die einen Gehalt an gebundener Milchsäure im Bereich von 20 bis 35 Gew.-% aufweisen.

## Claims

1. The use of lactic acid esters corresponding to formulae **(Ia), (Ib)** and/or **(Ic):** 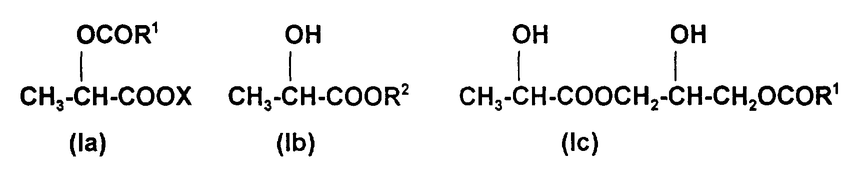 in which R¹CO is a linear or branched, saturated and/or unsaturated acyl group containing 6 to 22 carbon atoms, R² is an alkyl and/or alkenyl group containing 6 to 22 carbon atoms and X is an alkali metal and/or alkaline earth metal,
as o/w emulsifiers for the production of cosmetic and/or pharmaceutical preparations, **characterized in that** the emulsifiers have a content of bound lactic acid of 10 to 40% by weight.

2. The use claimed in claim 1, **characterized in that** lactic acid esters corresponding to formula (la), in which the acyl group R¹CO is derived from fatty acids containing 16 to 22 carbon atoms, are used.

3. The use claimed in claims 1 and 2, **characterized in that** lactic acid esters corresponding to formula (Ib), in which the alkyl group R² is derived from fatty alcohols containing 16 to 22 carbon atoms, are used.

4. The use claimed in claims 1 to 3, **characterized in that** lactic acid esters with a content of bound lactic acid of 20 to 35% by weight are used.

## Revendications

1. Utilisation d'esters d'acide lactique de formules Ia, Ib et/ou Ic, 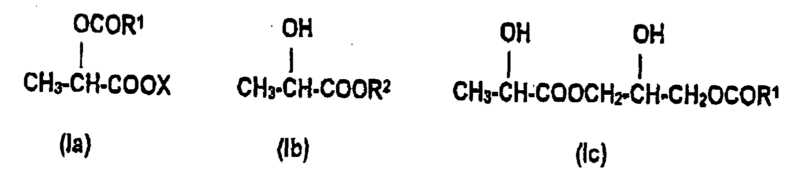 dans lesquelles R¹CO représente un reste acyle linéaire ou ramifié, saturé et/ou non saturé, ayant de 6 à 22 atomes de carbone, R² représente un reste alkyle et/ou alkényle ayant de 6 à 22 atomes de carbone, et X représentent un métal alcalin et/ou alcalino-terreux, comme agents émulsionnants H/E en vue de la production de préparations cosmétiques et/ou pharmaceutiques
**caractérisé en ce que**
les agents émulsionnants possèdent une teneur en acide lactique lié, dans la zone de 10 à 40 % en poids.

2. Utilisation d'esters d'acide lactique selon la revendication 1,
**caractérisée en ce qu'**
on met en oeuvre des esters d'acide lactique de formule (Ia) dans lesquels le reste acyle R¹CO dérive d'acides gras ayant de 16 à 22 atomes de carbone.

3. Utilisation selon d'esters d'acide lactique les revendications 1 et 2,
**caractérisée en ce qu'**
on met en oeuvre des esters d'acide lactique de formule (Ib) pour lesquels le reste acyle R² dérive d'alcools gras ayant de 16 à 22 atomes de carbone.

4. Utilisation d'esters d'acide lactique selon les revendications 1 à 3,
**caractérisée en ce qu'**
on met en oeuvre des esters d'acide lactique qui possèdent une teneur en acide lactique lié dans la zone de 20 à 35 % en poids.
